# EUROPEAN PATENT APPLICATION

(11) **EP 3 109 323 A1**
(43) Date of publication of application: **28.12.2016**
(21) Application number: 16726760.8
(22) Date of filing: 15.03.2016
(51) Int. Cl.: C12Q 1/24, C12N 5/09, C12M 1/12

(54) **TARGET CELL COLLECTION METHOD**

(30) Priority: 30.04.2015 KR 20150061091
(71) Applicant: Cytogen Co., Ltd., Songpa-gu Seoul 05839 (KR)
(72) Inventor: JEON, Byung Hee, Seongnam-si Gyeonggi-do 13611 (KR); HAN, Sang Soo, Seocho-gu Seoul 06744 (KR)
(74) Representative: Zardi, Marco
(86) International application number: PCT/KR2016/002545
(87) International publication number: WO 2016/175451

(57) **Abstract**

[Problem to be Solved]

Provided is a method of collecting target cells capable of separating target cells from a fluid sample including blood and physiological fluid, and the like and efficiently collecting target cells while preventing the damage to the separated target cells.

[Solution]

The method of collecting the target cells that are separated from a cell mixed solution, the method comprising: moving a biochip positioned with the separated target cells into a container; firstly injecting a buffer into the biochip; and collecting the target cells in a collection space by firstly absorbing the buffer in a first region in the biochip.

## Description

### [Technical Field]

The present invention relates to a method of collecting target cells. More particularly, to a method of collecting target cells capable of separating target cells from a fluid sample including blood and physiological fluid, and the like and efficiently collecting target cells while preventing the damage to the separated target cells.

### [Background Art]

Recently, regulations on an animal test and a clinical test for treating human diseases have been reinforced. In order to replace the animal test and the clinical test, development of researches and techniques for collecting live cells from the human blood have been actively conducted. The collecting of the cells is performed by various collecting devices including a microfluidic device, a circulating tumor cells (CTC) chip, a filter, and the like.

As an example of the cell collecting device, in U.S Patent application publication No. 2007/0025883A1, a parylene membrane filter for filtering cells from a fluid is provided. The membrane filter is installed in a chamber and has a plurality of pores formed to prevent cell, for example, cancer cells from being passed.

As another example of the cell collecting device, in US Patent application publication No. 2009/0188864A1, a method and an apparatus for microfiltration to perform cell separation are provided. A plurality of filter patches is installed in a central square hole of the apparatus for microfiltration. The filter patches are constituted by a membrane having a plurality of pores for filtering the cells. However, in the techniques of the patent documents, in order to recover and collect cancer cells filtrated in the filter from the chamber or the central square hole, since the cancer cells need to be forcibly discharged outside the chamber by supplying a solution, for example, water in the chamber backwards, it is difficult to recover and collect the cancer cells from the filter. Further, there is a problem in that the cancer cells are easily damaged in the discharging process from the chamber.

Accordingly, a method of collecting target cells capable of separating target cells from a fluid sample including blood and physiological fluid, and the like and efficiently collecting target cells while preventing the damage to the separated target cells is required.

### [Summary of Invention]

### [Technical Problem]

An object of the present invention is to separate target cells from a fluid sample including blood and physiological fluid, and the like and efficiently collect target cells while preventing the damage to the separated target cells.

Technical objects to be achieved in the present invention is not limited to the aforementioned technical objects, and other not-mentioned technical objects will be obviously understood by those skilled in the art from the description below.

### [Solution to Problem]

An aspect of the present invention provides a method of collecting the target cells that are separated from a cell mixed solution, the method comprising: moving a biochip positioned with the separated target cells into a container; firstly injecting a buffer into the biochip; and collecting the target cells in a collection space by firstly absorbing the buffer in a first region in the biochip. The method has advantages of separating the target cells from a fluid sample including blood and physiological fluid, and the like and efficiently collecting target cells while preventing the damage to the separated target cells.

### [Advantageous Effects of Invention]

The present invention has the following effect.

According to the present invention, it is possible to separate target cells from a fluid sample including blood and physiological fluid, and the like and efficiently collect the target cells while preventing the damage to the separated target cells.

### [Brief Description of Drawings]

[Figure 1] Figure 1 is a diagram for describing a structure of a biochip.
[Figure 2] Figure 2 is a diagram for describing an appearance in which target cells are separated from a cell mixed solution by using the biochip.
[Figure 3] Figure 3 is a flowchart of a method of collecting target cells according to the present invention.
[Figure 4] Figure 4 is a diagram for describing Example of the method of collecting the target cells according to the present invention.
[Figure 5] Figure 5 is a diagram for describing Comparative Example of the method of collecting the target cells according to the present invention.
[Figure 6] Figure 6 is a graph for Example and Comparative Example of the method of collecting the target cells according to the present invention.
[Figure 7] Figure7 is an experimental data value representing excellence of the method of collecting the target cells according to the present invention.

### [Description of Embodiments]

In order to achieve the object, a method of collecting target cells which are separated from a cell mixed solution according to the present invention includes moving a biochip positioned with the separated target cells into a container, firstly injecting a buffer into the biochip, and collecting a collection space by firstly absorbing the buffer in a first region in the biochip.

In this case, in the moving into the container, the biochip moves into the container including the buffer, and in the firstly injecting, the buffer included in the container is injected into the biochip through a mesh at the lower end of the biochip.

In this case, in the moving into the container, the biochip moves into the container without including the buffer, and the firstly injecting includes injecting the buffer into the container and injecting the buffer included in the container into the biochip through a mesh at the lower end of the biochip.

In this case, in the firstly injecting, a buffer included in the container or a second container which is another container other than the container is injected into the biochip.

In this case, in the firstly injecting, the buffer climbs on the inner wall of the biochip and falls down the inside of the biochip by injecting the buffer into the inner wall of the biochip which is a lateral direction of the inside of the biochip.

In this case, after collecting the target cells in the collection space by firstly absorbing the buffer, the method further includes secondly injecting the buffer into the biochip and collecting the target cells in the collection space by secondly absorbing the buffer in a second region in the biochip.

In this case, after collecting the target cells in the collection space by secondly absorbing the buffer, the method further includes collecting the target cells in the collection space by thirdly absorbing the buffer in a third region in the biochip.

In this case, after collecting the target cells in the collection space by thirdly absorbing the buffer, the method further includes collecting the target cells in the collection space by fourthly absorbing the buffer in a fourth region in the biochip.

In this case, the biochip may be constituted by a sleeve and a chip coupled with a lower end of the sleeve.

In this case, the first region is constituted by a plurality of first subregions which is spaced apart from each other at the same or different intervals along a first outer side which is the outermost side of the lower end of the biochip, and in the collecting of the target cells in the collection space by firstly absorbing the buffer in the first region, the target cells are collected in the collection space by circulating the buffer in the plurality of first subregions in a clockwise or a counterclockwise and absorbing the buffer.

In this case, the second region is constituted by a plurality of second subregions which is spaced apart from each other at the same or different intervals along a second outer side which is the outer side formed in the first outer side, and in the collecting of the target cells in the collection space by secondly absorbing the buffer in the second region, the target cells is collected in the collection space by circulating the buffer in the plurality of second subregions in a clockwise or a counterclockwise and absorbing the buffer.

In this case, the third region is constituted by a plurality of third subregions which is spaced apart from each other at the same or different intervals along a third outer side which is the outer side formed in the second outer side, and in the collecting of the target cells in the collection space by thirdly absorbing the buffer in the third region, the target cells is collected in the collection space by circulating the buffer in the plurality of third subregions in a clockwise or a counterclockwise and absorbing the buffer.

In this case, the fourth region is a center of the lower end of the biochip, and in the collecting of the target cells in the collection space by fourthly absorbing the buffer in the fourth region, the target cells are collected in the collection space by absorbing the buffer in the fourth region.

In this case, in the collecting of the target cells in the collection space by firstly absorbing the buffer, the buffer may be absorbed while a distance between the lower end of the biochip and an end of the absorbing means for absorbing the buffer is maintained to be 0.02 mm to 1.0 mm.

In this case, the target cells may be circulating tumor cells (CTSs).

### [Embodiments]

The present invention may be variously modified and have various exemplary embodiments, so that specific embodiments will be illustrated in the drawings and described in the detailed description.

However, this does not limit the present invention within specific exemplary embodiments, and it should be understood that the present invention covers all the modifications, equivalents and replacements within the idea and technical scope of the present invention. In the description of each drawing, similar reference numerals are used for similar constitute elements.

It should be understood that, when it is described that an element is "coupled" or "connected" to another element, the element may be "directly coupled" or "directly connected" to the another element or "coupled" or "connected" to the another element through a third element. In contrast, it should be understood that, when it is described that an element is "directly coupled" or "directly connected" to another element, it is understood that no element is not present between the element and the another element.

Terms used in the present application are used only to describe specific exemplary embodiments, and are not intended to limit the present invention. Singular expressions used herein include plurals expressions unless they have definitely opposite meanings. In the present application, it should be understood that term "include" or "have" indicates that a feature, a number, a step, an operation, a component, a part or the combination thereof described in the specification is present, but does not exclude a possibility of presence or addition of one or more other features, numbers, steps, operations, components, parts or combinations, in advance.

Hereinafter, preferred exemplary embodiments of the present invention will be described in more detail with reference to the accompanying drawings. Hereinafter, like constituent elements uses like reference numerals in the drawings and the duplicated description of the like constituent elements will be omitted.

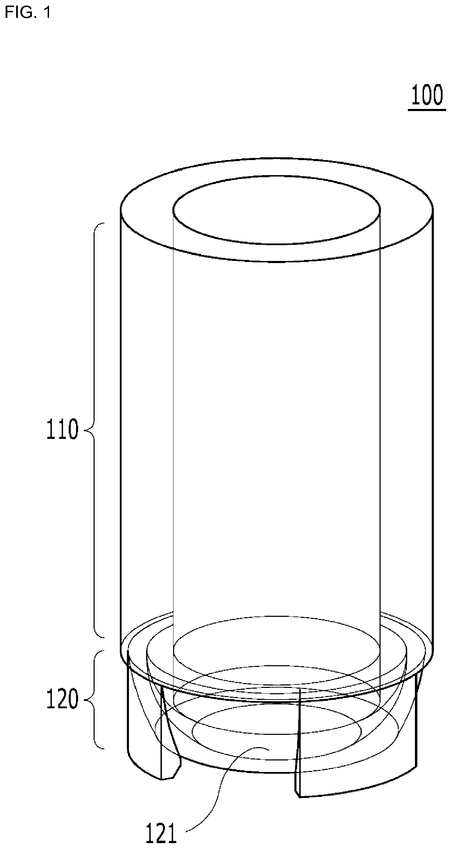

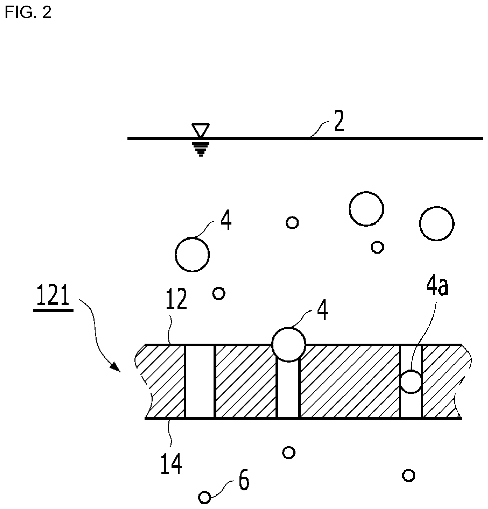

Figure 1 is a diagram for describing a structure of a biochip. Figure 2 is a diagram for describing an appearance in which target cells are separated from a cell mixed solution by using the biochip.

The target cells in the present invention mean preferably circulating tumor cells (CTCs) and are not necessarily limited to the CTCs. The CTCs are known as a main factor of metastasis as a small number of tumor cells which are separated from a primary tumor tissue, that is, primary cancer to be circulated in the blood. According to statistics, it is examined that approximately 90% of cancer patients are died due to the metastasis.

The CTCs reach one to several thousand per 1 ml of the blood according to a kind of cancer, and recently, various attempts capable of efficiently separating the CTCs have been conducted.

The collection of the cells is performed by various cell collecting devices including a biochip, a microfluidic device, and the like. The biochip is classified into a microarray and a microfluidic device. The microarray is a device of capturing and analyzing a target including DNA, protein, enzyme, and the like from a sample including a physiological fluid including human saliva, perspiration, and the like, the blood, and the like by aligning deoxyribonucleic acids (DNAs), proteins, and the like, and includes a DNA chip, a protein chip, and the like.

The microfluidic device is a device of analyzing a target that reacts with a sensor, biomolecules, and the like while flowing the sample, and is also referred to as a microfluidic chip or Lap-on a chip.

Referring to Figure 1, a biochip 100 is constituted by a sleeve 110 and a chip 120 and the method of collecting the target cells according to the present invention may be implemented while the sleeve 110 and the chip 120 are separated from or coupled with each other. In this case, a height of the sleeve 110 is sufficient enough not to flow the buffer in the container into the biochip 100, and the shape is not limited to the exemplary embodiment illustrated in Figure 1 and may be variously modified. Further, a lower end 121 of the chip 120 has a plurality of pores for filtering the target cells.

Referring to Figure 2, in more detail, a blood (cell mixed solution) 2 includes a plurality of cancer cells 4 as target cells and a plurality of non-target cells 6 in order to filter the target cells by the lower end 121 of the chip 120 having the plurality of pores. The non-target cells 6 are constituted by red blood cells and white blood cells.

The lower end 121 has a surface 12, a rear surface 14, and a plurality of filtering holes formed for filtering the cancer cells 4. The filtering holes are arranged in a regular pattern. Further, the shape of the lower end 121 may be appropriately modified to a square, an oval, and the like, but it will be understood that a preferred shape for implementing the method of collecting the target cells according to the present invention is a circle.

Diameters of the filtering holes are smaller than diameters of the cancer cells 4. The diameters of the filtering holes are formed with 5 to 25 µm. Preferably, the diameters of the filtering holes may be formed with 7 to 25 µm. The lower end 121 may be prepared with a material such as stainless steel, nickel, aluminum, and copper. The filtering holes having micrometer sizes may be formed by etching using micro-electro mechanical systems (MEMS).

The blood (cell mixed solution) 2 is supplied to the biochip 100 by a blood supplying means. The blood supplying means may be constituted by various forms including a syringe, a blood collection tube, a bag, a pack, and the like in which a required amount of blood is stored to be supplied to the biochip. Further, the blood supplying means may be constituted by a syringe pump, a plunger pump, and the like.

The cancer cells 4 in the blood (cell mixed solution) 2 do not pass through the filtering holes and remain on the surface 12. The cancer cells 4 do not pass through the filtering holes having small diameters due to a lower deformation rate than the red blood cells and the white blood cells.

Meanwhile, the non-target cells 6 pass through the filtering holes. As an example of the non-target cells 6, a diameter of the red blood cell is approximately 7.2 to 8.4 µm. The human red blood cell has a concave disk shape and has no nucleus. The red blood cells easily pass through the filtering holes having diameters of 5 µm due to a high deformation rate. When the diameters of the filtering holes are formed with 7 µm so as to filter the cancer cells 4 having the diameters of 7 µm or more, the red blood cells smoothly pass through the filtering holes. Accordingly, a clogging phenomenon of the filtering holes due to approximately 5 million of red blood cells per 1 mm³ blood is prevented and the flow of the blood 2 is smoothly maintained.

As another example of the non-target cells 6, kinds of white blood cells are various according to shapes and sizes thereof, shapes and the number of nucleus, and particles or not and the diameter thereof is approximately 10 to 21 µm. The white blood cells easily pass through the filtering holes having smaller diameters than the diameters of the white blood cells due to deformation of cytoplasma. A diameter of the largest macrophage of the white blood cells is 21 µm. When the diameters of the filtering holes are formed with 20 µm, most of 7,000 to 8,000 white blood cells per 1 mm³ blood pass through the filtering holes 16.

As such, when the non-target cells 6 pass through the filtering holes, most of the target cells (cancer cells) and a small amount of non-target cells are present in the biochip 100.

Herein, the method for collecting the target cells (cancer cells) remaining in the biochip 100 is proposed.

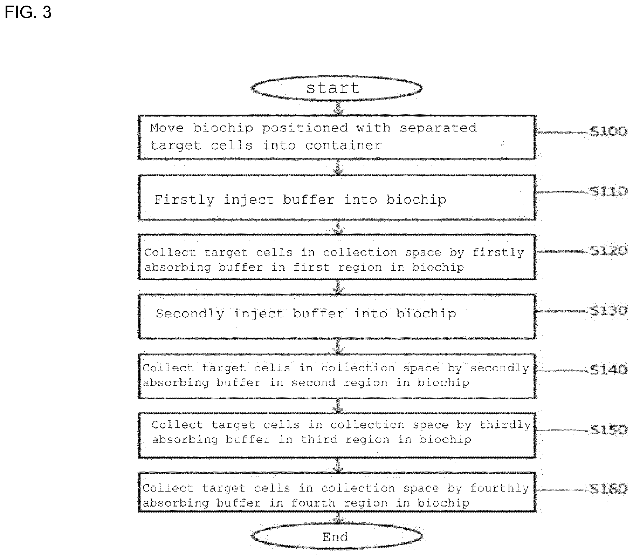

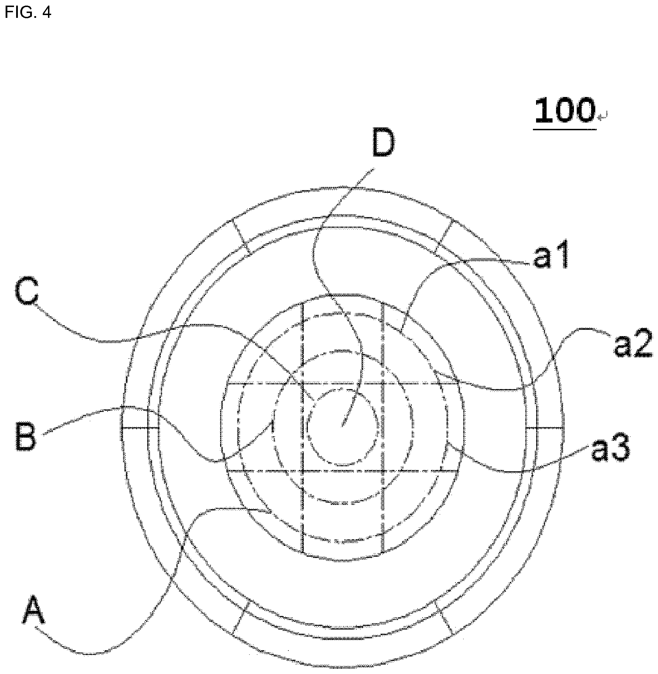

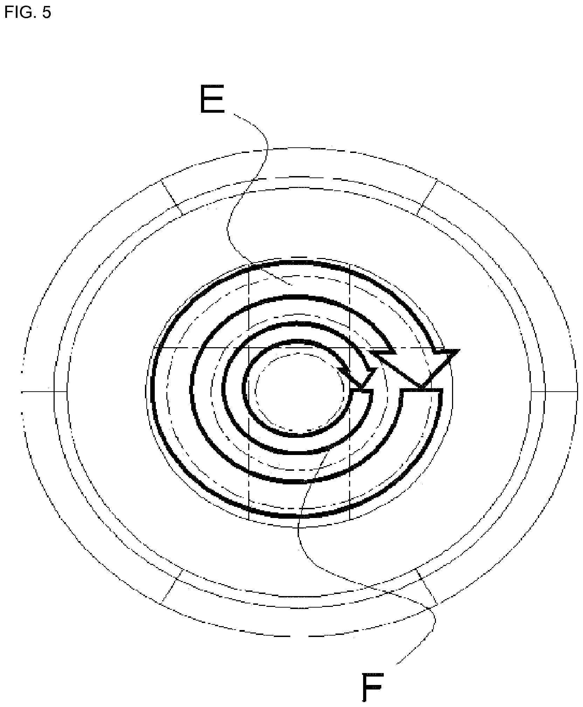

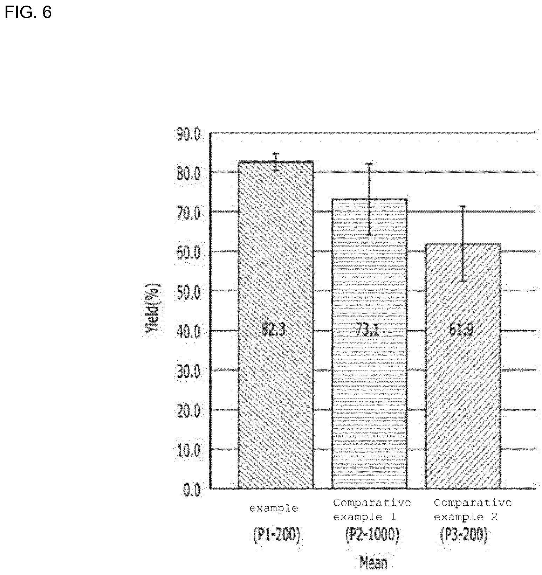

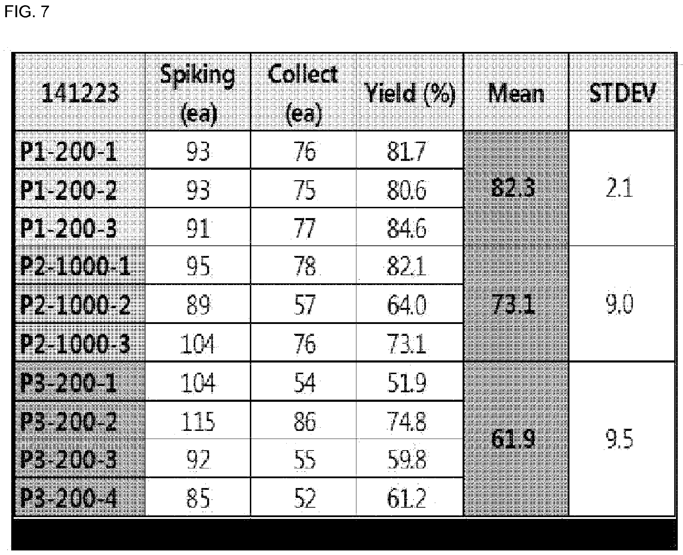

Figure 3 is a flowchart of a method of collecting target cells according to the present invention. Figure 4 is a diagram for describing Example of the method of collecting the target cells according to the present invention. Figure 5 is a diagram for describing Comparative Example of the method of collecting the target cells according to the present invention. Figure 6 is a graph for Example and Comparative Example of the method of collecting the target cells according to the present invention. Figure7 is an experimental data value representing excellence of the method of collecting the target cells according to the present invention.

Referring to Figure 3, when describing the exemplary embodiment of the present invention, the method of collecting the target cells that are separated from the cell mixed solution according to the present invention includes moving a biochip positioned with the separated target cells to a container (S100), firstly injecting a buffer into the biochip (S110), collecting the target cells in a collection space by firstly absorbing the buffer in a first region in the biochip (S120), secondly injecting the buffer into the biochip (S130), collecting the target cells in the collection space by secondly absorbing the buffer in a second region in the biochip (S140), collecting the target cells in the collection space by thirdly absorbing the buffer in a third region in the biochip(S150), and collecting the target cells in the collection space by fourthly absorbing the buffer in a fourth region in the biochip.

In more detail, in relation to the exemplary embodiment of step S100, the biochip may be moved into the container with the buffer and may also be moved to the container without the buffer.

Further, in relation to the exemplary embodiment of step S110, as a method of injecting the buffer into the biochip, the buffer included in the container may be injected into the biochip and the buffer included in a second container other than the container may also be injected into the biochip. Further, as the method of injecting the buffer into the biochip, a method of directly injecting the buffer from the container or the second container may be performed. The buffer included in the container may also be injected into the biochip through a mesh formed at the lower end of the biochip by inserting the biochip to the container with the buffer.

Further, as the method of injecting the buffer into the biochip, multiple times of injection may be performed by appropriately selecting or combining the methods. That is, it should be understood that the buffer may be additionally injected through different methods or the same method.

In relation to the method of directly injecting the buffer included in the container or the second container into the biochip, according to the exemplary embodiment, the buffer may climb on the inner wall of the biochip to fall down into the biochip by injecting the buffer into the inner wall of the biochip which is the lateral direction in the biochip. As such, the buffer is not injected vertically to the lower end of the biochip, but injected along the side wall of the biochip to prevent the damage to the target cells.

Further, the first region is constituted by a plurality of first subregions which is spaced apart from each other at the same or different intervals along a first outer side which is the outermost side of the lower end of the biochip, the second region is constituted by a plurality of second subregions which is spaced apart from each other at the same or different intervals along a second outer side which is an outer side formed in the first outer side, the third region is constituted by a plurality of third subregions which is spaced apart from each other at the same or different intervals along a third outer side which is an outer side formed in the second outer side, and the fourth region means a center of the lower end of the biochip. In more detail, the shape of the lower end of the biochip may be a circle, and according to an exemplary embodiment, the shape may be constituted by a rectangular or triangular shape. However, since the first region is formed outside the second region, the number of first subregions may be larger than the number of second subregions. Since the second region is formed outside the third region, the number of second subregions may be larger than the number of third subregions.

Further, in the colleting of the buffer in the collecting space by firstly absorbing the buffer (S120), the collecting of the target cells in the collecting space by secondly absorbing the buffer (S140), the collecting of the target cells in the collecting space by thirdly absorbing the buffer (S150), and the collecting of the target cells by fourthly absorbing the buffer (S160), the buffer may be absorbed while a distance between the lower end of the biochip and the end of the absorbing means for absorbing the buffer is maintained with 0.02 mm to 1.0 mm. When the buffer is absorbed with the distance of less than 0.02 mm, since the distance between the end of the absorbing means (a pipette or the like) and the lower end of the biochip is very short, the damage to the target cells may occur. When the buffer is absorbed with the distance of more than 1.0 mm, the efficient absorption is not performed.

Referring to Figure 4, when describing the actually experimented contents corresponding to each step in detail, in step S100, a biochip (sleeve + chip) after washing moves to a container of 35 mm including the buffer and in step S110, the buffer in the container is put into the biochip by 70 µl. In this case, when the buffer is injected vertically to the lower end of the biochip, the damage to the target cells may occur and thus, the buffer is injected toward the side wall of the biochip.

Thereafter, in relation to step S120, in the collecting of the target cells in the collection space by firstly absorbing the buffer in a first region A in the biochip, the first region A is constituted by first subregions a1, a2, a3, ... which are spaced apart from each other at the same interval along a circumference of the lower end of the biochip, and the target cells are collected in the collection space by circulating the 16 first subregions by 50 µl in a clockwise or counterclockwise and absorbing the buffer through a pipette.

Thereafter, in relation to step S130, 700 µl of the buffer is secondly injected into the biochip, and in the relation to step S140, in the collecting of the target cells in the collection space by secondly absorbing the buffer in the second region B, the target cells are collected in the collection space by circulating the 8 second subregions by 50 µl in a clockwise or counterclockwise and absorbing the buffer through the pipette.

Further, in relation to step S150, the third region C is constituted by four third subregions which are spaced apart from each other at the same interval along the circumference of the lower end of the biochip between the second region B and the center of the lower end of the biochip. In addition, in the collecting of the target cells in the collection space by thirdly absorbing the buffer in the third region C in the biochip, the target cells are collected in the collection space by circulating the four third subregions by 50 µl in a clockwise or counterclockwise and absorbing the buffer through the pipette.

Further, in relation to step S160, in the collecting of the target cells in the collection space by fourthly absorbing the buffer in the fourth region D in the biochip, the target cells are collected in the collection space by absorbing 100 µl of the buffer through the pipette in the fourth region. The collection space has 24 wells and a total of 1.5 ml of the target cells are collected through the above steps. The exemplary embodiment of the present invention is performed three times and the result thereof will be described below.

Comparative Examples for comparing Example of the method of collecting the target cells according to the present invention are performed by two types as described below.

Referring to Figure 5, Comparative Examples 1 and 2 will be described.

When describing Comparative Example 1 in sequence with reference to Figure 5,
1. Sleeve + Chip moves to a container of 35 mm including 3.5 ml of a buffer.
2. 700 µl of the buffer in the container is firstly injected into the chip.
3. 700 µl of the buffer is firstly absorbed from a large circle E to a small circle F after mixing three times.
4. 700 µl of the buffer in the container is secondly injected into the chip.
5. 700 µl of the buffer is secondly absorbed from the large circle E to the small circle F after mixing three times.

When the first absorption and the second absorption are performed as described above, 1.5 ml of the target cells are collected in the 24 wells which are the collection space.

When describing Comparative Example 2 in sequence with reference to Figure 5,
1. Sleeve + Chip moves to a container of 35 mm including 3.5 ml of a buffer.
2. 700 µl of the buffer in the container is firstly injected in to the chip.
3. A total of 750 µl of the buffer is firstly absorbed by absorbing 150 µl of the buffer in the small circle F after absorbing 150 µl of the buffer in the large circle E five times.
4. 700 µl of the buffer in the container is secondly injected into the chip.
5. A total of 750 µl of the buffer is secondly absorbed by absorbing 150 µl of the buffer in the small circle F after absorbing 150 µl of the buffer in the large circle E five times.

When the first absorption and the second absorption are performed as described above, 1.5 ml of the target cells are collected in the 24 wells which are the collection space.

Figure 6 is a graph for Example and Comparative Example of the method of collecting the target cells according to the present invention. Referring to Figure 6, it can be seen that Example P1-200 of the method of collecting the target cells according to the present invention has a higher collection rate than Comparative Example 1 P2-1000 and Comparative Example 2 P3-200.

Figure 7 is an experimental data value representing excellence of the method of collecting the target cells according to the present invention. As illustrated in Figure 7, it can be seen that the collection rate of Examples P1-200-1, P1-200-2, and P1-200-3 of the method of collecting the target cells according to the present invention is more improved than that of Comparative Examples 1 P2-1000-1, P2-1000-2, and P2-1000-3 and Comparative Examples 2 P3-200-1, P3-200-2, P3-200-3, P3-200-4.

The present description presents a best mode of the present invention and provides examples for describing the present invention and so that those skilled in the art easily manufacture and use the present invention. In the prepared specification, the present invention is not limited to the presented detailed terms.

Accordingly, the present invention has been described in detail with reference to the aforementioned examples, but modifications, changes, and transformations of the examples can be made by those skilled in the art without departing from the scope of the present invention. For example, all functions illustrated in the drawings need not be separately included or all orders illustrated in the drawings need not just be followed in the illustrated order in order to achieve intended by the present invention and otherwise, it should be noted that the modifications, changes, and transformations of the examples can be included in the technical scope of the present invention disclosed in the claims in any degree.

## Claims

1. A method of collecting target cells that are separated from a cell mixed solution, the method comprising:
moving a biochip positioned with the separated target cells into a container;
firstly injecting a buffer into the biochip; and
collecting the target cells in a collection space by firstly absorbing the buffer in a first region in the biochip.

2. The method of claim 1, wherein in the moving of the biochip into the container, the biochip is moved into the container including the buffer, and in the first injection, the buffer included in the container is injected into the biochip through a mesh at the lower end of the biochip.

3. The method of claim 1, wherein in the moving of the biochip into the container, the biochip is moved into the container without including the buffer and the first injection includes injecting the buffer into the container; and injecting the buffer included in the container into the biochip through a mesh at the lower end of the biochip.

4. The method of claim 1, wherein in the first injecting, the buffer included in the container or a second container which is another container other than the container is injected into the biochip.

5. The method of claim 1, wherein in the first injecting, the buffer climbs on the inner wall of the biochip to fall down into the biochip by injecting the buffer into the inner wall of the biochip which is a lateral direction in the biochip.

6. The method of claim 1, further comprising:
secondly injecting the buffer into the biochip after the collecting the target cells in the collection space by firstly absorbing the buffer; and
collecting the target cells in the collection space by secondly absorbing the buffer in a second region in the biochip.

7. The method of claim 6, further comprising:
collecting the target cells in the collection space by thirdly absorbing the buffer in a third region in the biochip after collecting the target cells in the collection space by secondly absorbing the buffer.

8. The method of claim 7, further comprising:
collecting the target cells in the collection space by fourthly absorbing the buffer in a fourth region in the biochip after collecting the target cells in the collection space by thirdly absorbing the buffer.

9. The method of claim 1, wherein the biochip is constituted by a sleeve; and a chip coupled with the lower end of the sleeve.

10. The method of claim 1, wherein the first region is constituted by a plurality of first subregions which is spaced apart from each other at the same or different intervals along a first outer side that is the outermost side of the lower end of the biochip, and
in the collecting of the target cells in the collection space by firstly absorbing the buffer in the first region, the target cells are collected in the collection space by circulating the plurality of first subregions in a clockwise or counterclockwise and absorbing the buffer.

11. The method of claim 6, wherein the second region is constituted by a plurality of second subregions which is spaced apart from each other at the same or different intervals along a second outer side that is an outer side formed in the first outer side, and
in the collecting of the target cells in the collection space by secondly absorbing the buffer in the second region, the target cells are collected in the collection space by circulating the plurality of second subregions in a clockwise or counterclockwise and absorbing the buffer.

12. The method of claim 7, wherein the third region is constituted by a plurality of third subregions which is spaced apart from each other at the same or different intervals along a third outer side that is an outer side formed in the second outer side, and
in the collecting of the target cells in the collection space by thirdly absorbing the buffer in the third region, the target cells are collected in the collection space by circulating the plurality of third subregions in a clockwise or counterclockwise and absorbing the buffer.

13. The method of claim 8, wherein the fourth region is a center of the lower end of the biochip, and
in the collecting of the target cells in the collection space by fourthly absorbing the buffer in the fourth region, the target cells are collected in the collection space by absorbing the buffer in the fourth region.

14. The method of claim 1, wherein in the collecting of the target cells collected in the collection space by firstly absorbing the buffer, the buffer is absorbed while a distance between the lower end of the biochip and an end of an absorbing means for absorbing the buffer is maintained to be 0.02 mm to 1.0 mm.

15. The method of claim 1, wherein the target cells are circulating tumor cells (CTCs).
